# EUROPEAN PATENT SPECIFICATION

(11) **EP 2 754 436 B1**
(45) Date of publication and mention of the grant of the patent: **16.05.2018**
(21) Application number: 13177251.9
(22) Date of filing: 19.07.2013
(51) Int. Cl.: A61K 9/00, A61K 39/12, A61K 47/51, A61P 31/14, C07K 14/18

(54) **Biodegradable high-efficiency dengue vaccine, method for making the same, and pharmaceutical composition comprising the same**
Hocheffizienter biologisch abbaubarer Dengue-Impfstoff, Herstellungsverfahren, pharmazeutische Zusammensetzung, die diesen enthält
Vaccin contre le virus de la dengue à rendement élevé biodégradable, son procédé de fabrication, et composition pharmaceutique le comprenant

(30) Priority: 09.01.2013 TW 102100790
(43) Date of publication of application: 16.07.2014
(73) Proprietor: National Cheng Kung University, Tainan City (TW)
(72) Inventor: Lin, Yee-Shin, TAINAN CITY (TW); Chen, Yu-Hung, TAINAN CITY (TW)
(74) Representative: Lang, Christian

(56) References cited:
- EP-A1- 2 428 221
- EP-A2- 2 446 899
- NIYATI KHETARPAL ET AL: "Dengue-specific subviral nanoparticles: design, creation and characterization", JOURNAL OF NANOBIOTECHNOLOGY, vol. 11, no. 1, 1 January 2013 (2013-01-01), pages 15-15, XP055067196, ISSN: 1477-3155, DOI: 10.1371/journal.pntd.0001735
- ELISÂNGELA F SILVA ET AL: "A tetravalent dengue nanoparticle stimulates antibody production in mice", JOURNAL OF NANOBIOTECHNOLOGY, BIOMED CENTRAL, GB, vol. 10, no. 1, 22 March 2012 (2012-03-22) , page 13, XP021125227, ISSN: 1477-3155, DOI: 10.1186/1477-3155-10-13

### SEQUENCE LISTING

<110> National Cheng Kung University
<120> Dengue Vaccine, Medicinal Composition Comprising the Same, Nucleotide Sequence, and Antibody Composition
<130> N/A
<160> 1
<170> PatentIn version 3.5
<210> 1
   <211> 352
   <212> PRT
   <213> Dengue Virus & Japanese Encephalitis Virus
<400> 1
   Asp Ser Gly Cys Val Val Ser Trp Lys Asn Lys Glu Leu Lys Cys Gly
   1               5                   10                  15
   Ser Gly Ile Phe Ile Thr Asp Asn Val His Thr Trp Thr Glu Gln Tyr
               20                  25                  30
   Lys Phe Gln Pro Glu Ser Pro Ser Lys Leu Ala Ser Ala Ile Gln Lys
           35                  40                  45
   Ala His Glu Glu Gly Ile Cys Gly Ile Arg Ser Val Thr Arg Leu Glu
       50                  55                  60
   Asn Leu Met Trp Lys Gln Ile Thr Pro Glu Leu Asn His Ile Leu Ser
   65                  70                  75                  80
   Glu Asn Glu Val Lys Leu Thr Ile Met Thr Gly Asp Ile Lys Gly Ile
                   85                  90                  95
   Met Gln Ala Gly Lys Arg Ser Leu Arg Pro Gln Pro Thr Glu Leu Lys
               100                 105                 110
   Tyr Ser Trp Lys Thr Trp Gly Lys Ala Lys Met Leu Pro Thr Glu Ser
           115                 120                 125
   His Asn Gln Thr Phe Leu Ile Asp Gly Pro Glu Thr Ala Glu Cys Pro
       130                 135                 140
   Asn Thr Asn Arg Ala Trp Asn Ser Leu Glu Val Glu Asp Tyr Gly Phe
   145                 150                 155                 160
   Gly Val Phe Thr Thr Asn Ile Trp Leu Lys Leu Arg Glu Lys Gln Asp
                   165                 170                 175
   Val Phe Cys Asp Ser Lys Leu Met Ser Ala Ala Ile Lys Asp Asn Arg
               180                 185                 190
   Ala Val His Ala Asp Met Gly Tyr Trp Ile Glu Ser Ala Leu Asn Asp
           195                 200                 205
   Thr Trp Lys Ile Glu Lys Ala Ser Phe Ile Glu Val Lys Ser Cys His
       210                 215                 220
   Trp Pro Lys Pro His Thr Leu Trp Ser Asn Gly Val Leu Glu Ser Glu
   225                 230                 235                 240
   Met Ile Ile Pro Lys Asn Phe Ala Gly Pro Val Ser Gln His Asn Tyr
                   245                 250                 255
   Arg Pro Gly Tyr His Thr Gln Thr Ala Gly Pro Trp His Leu Asn Gly
               260                 265                 270
   Ile Val Leu Asp Phe Asp Tyr Cys Pro Gly Thr Lys Val Thr Ile Thr
           275                 280                 285
   Glu Asp Cys Gly Lys Arg Gly Pro Ser Val Arg Thr Thr Thr Asp Ser
       290                 295                 300
   Gly Lys Leu Ile Thr Asp Trp Cys Cys Arg Ser Cys Ser Leu Pro Pro
   305                 310                 315                 320
   Leu Arg Phe Arg Thr Glu Asn Gly Cys Trp Tyr Gly Met Glu Ile Arg
                   325                 330                 335
   Pro Val Arg His Asp Glu Thr Thr Leu Val Arg Ser Gln Val Asp Ala
               340                 345                 350

## Description

### BACKGROUND OF THE INVENTION

### 1. Field of the Invention

The present invention relates a biodegradable high-efficiency dengue vaccine. More particularly, a biodegradable nanocomplex with electric property holds a dengue viral protein inside, after vaccination with the foregoing biodegradable nanocomplex twice, an organism has an antibody response, and the antibody production is increased widely for inducing immune responses efficiently.

### 2. Description of Related Art

Dengue fever, also known as breakbone fever, is an acute viral disease transmitted by *Adeds aegypti* or *Aedes albopictus,* and its symptoms include fever (39 °C - 40 °C) or aversion to cold, skin rash with fatigue in limb, muscle pains, frontal headache, orbital pain, abdominal pain, backache (i.e. the origin of the term "breakbone fever"), sore throat, and maybe vomiting, fainting, etc.. The commonly mentioned dengue fever is classic dengue fever, also called as primary dengue fever. In addition, severe and life-threatening dengue fever characterized by hemorrhage or shock may be developed, also called dengue hemorrhagic fever (DHF) or dengue shock syndrome (DSS), or secondary dengue fever. It is estimated that there are about 50 million to 100 million cases of dengue infection worldwide each year, with about 250,000 to 500,000 cases of dengue hemorrhagic fever. Hence, the prevention and treatment of dengue fever is an important issue for the governments of many countries. Since dengue virus is the major pathogen of dengue disease, the early detection or prevention with effective vaccine can efficiently control morbidity and death rates of dengue fever.

Please refer to the Taiwan Patent Publication No. 201210614 "Dengue vaccine, medicinal composition comprising the same, and nucleotide sequence" and the Taiwan Patent Publication No. 201210615 "Dengue vaccine, medicinal composition comprising the same, nucleotide sequence, and antibody composition", which are applied by the inventors of the present invention. A dengue vaccine causing no autoimmunity to avoid the cross-reaction between endothelial cell and platelets and being able to shorten the bleeding time is disclosed in the foregoing applications. The foregoing dengue vaccine has prospective effect in actual operation, but there is deficiency due to the aluminum hydroxide gel (also called as immunostimulant) as an adjuvant in the foregoing dengue vaccine. The ability of the aluminum hydroxide is undesired to enhance the immune response in the organism.

Commonly, the adjuvant action mechanism generally comprises: (a) increasing the life or the immunity of an antigen in the vaccine, (b) delivering antigen to the antigen-presenting cell, (c) improving antigen display in antigen-presenting cell, and (d) inducing the production of immunoregulatory cytokine. The mineral adjuvant is one of the common adjuvant, such as metal salts of zinc, calcium, cerium, chromium, iron, and beryllium. The aluminum salts, such as aluminum hydroxide and aluminum phosphate, is the most commonly used, and is also called as Alum adjuvant. The mechanism of the Alum adjuvant refers to the antigen being absorbed on the aluminum salt, which is also used as the immunostimulant, and when the antigen is taken up by antigen-presenting cell, the immunostimulant absorbed by the antigen stimulates the antigen-presenting cell at the same time.

Please refer to the US Patent No. 7357963. It disclosed a process for the manufacture of a vaccine, in which an adjuvant composition containing an immunostimulant adsorbed onto a first metallic salt particle substantially free of antigen is mixed with an antigen adsorbed onto a second metallic salt particle. The antigen is derived from human immunodeficiency virus, varicella zoster virus, human cytomegalovirus, dengue virus, hepatitis A, B, C or E virus. Actually, the Alum adjuvant is applied to over 50% of the commercial vaccine product, including hepatitis B vaccine (Alum-HBsAg), diphtheria and tetanus toxoid vaccine (Alum-DT), etc.. The foregoing antigen-metal complex vaccine is used for years and it is proved that the complex is absorbed easily by the antigen-presenting cell, but it is doubted that the safety of the heavy metal. Accordingly, the safety adjuvant used in the vaccine application should be developed to avoid the unsafe problems resulting from the Alum adjuvant and to enhance the antibody production for better immune responses and lowering the vaccination times and the cost.

EP 2 428 221 A1 refers to a dengue vaccine including chimeric nonstructural protein 1, which comprises the N-terminus of DV NS1 from amino-acid residues 1 to 270 and C-terminus of JEV NS1 from amino acid residues 271 to 352 (designated DJ NS1).

EP 2 446 899 A1 discloses an adjuvant composition containing poly-gamma-glutamic acid-chitosan nanoparticles and a vaccine composition containing the adjuvant composition useful for the treatment of viral and bacterial infections and cancers. By mixing a poly-gamma-glutamic acid solution and a chitosan solution with each other at a ratio of 1:1-8:1 (poly-gamma-glutamic acid: chitosan), poly-gamma-glutamic acid-chitosan nanoparticles having a negatively charged surface are prepared.

### SUMMARY OF THE INVENTION

In view of the foregoing disadvantages of the traditional dengue vaccine in actual operation, the object of the present invention is to provide a biodegradable high-efficiency dengue vaccine, comprising a biodegradable nanocomplex with an electric property holding a dengue viral protein inside, wherein the dengue viral protein is held in a positively charged biodegradable nanocomplex.

Accordingly, a biodegradable high-efficiency dengue vaccine is provided, which comprises a biodegradable nanocomplex with an electric property holding a dengue viral protein inside, wherein the biodegradable nanocomplex is made from a chitosan with positive charge and a polyglutamic acid with negative charge and the biodegradable nanocomplex having an electric property of positive charge, wherein a charge ratio of the chitosan to the polyglutamic acid is 4:1, and wherein the dengue viral protein comprises a nonstructural chimeric protein DJ NS1 of SEQ.ID. NO:1.

The nonstructural chimeric protein DJ NS1 comprises N-terminal amino acid 1-270 of a dengue virus nonstructural protein (DV NS1) and C-terminal amino acid 271-352 of a Japanese encephalitis virus nonstructural protein (JEV NS1). According to another embodiment of the present invention, the dose of the biodegradable nanocomplex is no more than 25 µg for the first vaccination. A method for making a biodegradable high-efficiency dengue vaccine is also provided. The method comprises the following steps. A dengue viral protein is added into a first solution to form a mixture solution, followed by adding said mixture solution into a second solution to form a biodegradable nanocomplex with positive charge by attraction force between the different electric properties, wherein the first solution comprises a first biodegradable macromolecule with a first electric property that is the same as an electric property of the dengue viral protein. According to the invention, the first electric property is a negative charge and the first biodegradable macromolecule is polyglutamic acid.. The second solution comprises a second biodegradable macromolecule with a second electric property that is opposite to the first electric property. According to the present invention, the second biodegradable macromolecule is chitosan. and a charge ratio of the chitosan to the polyglutamic acid is 4:1, and the dengue viral protein is held in the biodegradable nanocomplex.

A pharmaceutical composition comprising the foregoing biodegradable high-efficiency dengue vaccine is also provided. The pharmaceutical composition comprises a biodegradable high-efficiency dengue vaccine as aforementioned or an addition salt thereof with a pharmaceutically acceptable base, and at least one pharmaceutically acceptable excipient and is used for producing a vaccine or a drug for treating or preventing hemorrhagic dengue fever or dengue shock syndrome.

### BRIEF DESCRIPTION OF THE DRAWINGS

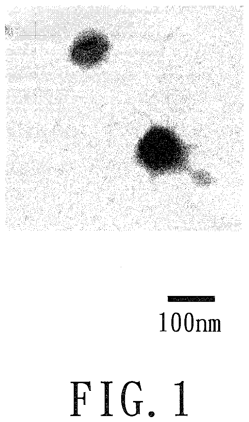

Fig. 1 is an electron microscope image of a biodegradable nanocomplex holding a dengue viral protein inside according to an embodiment of the present invention.

### DETAILED DESCRIPTION OF THE PREFERRED EMBODIMENT

A biodegradable high-efficiency dengue vaccine is disclosed, which comprises a biodegradable nanocomplex with electric properties holding a dengue viral protein inside. After vaccination with the biodegradable nanocomplex twice, an organism has antibody responses. The dose of the biodegradable nanocomplex is no more than 25 µg for the first vaccination. It is noted that the foregoing dengue viral protein can be dengue envelope protein or dengue nonstructural protein. According to an embodiment of the present invention, the dengue viral protein is disclosed in the Taiwan Patent Publication No. 201210614 "Dengue vaccine, medicinal composition comprising the same, and nucleotide sequence". The dengue viral protein comprises a nonstructural chimeric protein DJ NS1. The nonstructural chimeric protein DJ NS1 comprises N-terminal amino acid 1-270 of a dengue virus nonstructural protein (DV NS1) and C-terminal amino acid 271-352 of a Japanese encephalitis virus nonstructural protein (JEV NS1). Moreover, according to the present invention, the dengue viral protein comprises a nonstructural chimeric protein DJ NS1 of SEQ.ID. NO:1..

The method for making a biodegradable high-efficiency dengue vaccine of the present invention, which allows an organism to have an antigen tilter of 1:256000 after the second vaccination. Accordingly, in comparison with the prior art, the dengue vaccine of the present invention sharply increases the antibody production to induce the immune responses efficiently for enhancing the protection effect of the vaccine, and it could be performed by the following examples to further show the range of the actual application:

### Example 1: Prepare a first solution comprising a first biodegradable macromolecule

The first solution comprises a first biodegradable macromolecule with first electric property, and the first biodegradable macromolecule is polyglutamic acid (γ-PGA) or heparin and the first electric property is negative charge, for example. In detail, a proper amount of polyglutamic acid was added into de-ionized water and stirred by electromagnetic stirrer until the polyglutamic acid was totally dissolved. Then, the sodium in the polyglutamic acid solution was removed by membrane dialysis. The dialysis process was accomplished at 4 °C for preventing bacterial growth. After dialysis, the polyglutamic acid solution was put at -20 °C for being totally frozen. Then, the water content of the frozen polyglutamic acid solution was removed by lyophilization to obtain the crystallized powder of the polyglutamic acid. The crystallized powder of the polyglutamic acid was stored in a sterilized tube and put in a moisture-proof box. Finally, a proper amount of crystallized powder of the polyglutamic acid was taken and dissolved in the de-ionized water in a desired concentration, which was the first solution comprising the first biodegradable macromolecule with negative charge.

However, one skilled in the art will readily recognize that the foregoing method for preparation of the first solution is one of embodiments. After reading and understanding the descriptions of the present invention, it will be obvious to those skilled in the art that various modifications may be made and not limited to the foregoing embodiment.

### Example 2: Prepare a mixture solution containing a dengue viral protein and the first solution

A dengue viral protein with the same electric property as the first biodegradable macromolecule was dissolved in the first solution to form a mixture solution with negative charge. The dengue viral protein was disclosed in the Taiwan Patent Publication No. 201210614 "Dengue vaccine, medicinal composition comprising the same, and nucleotide sequence." The dose of the dengue viral protein was 100□ µg, 200□ µg, or 400 µg, and it is not limited here.

### Example 3: Prepare a second solution comprising a second biodegradable macromolecule

A second solution comprising a second biodegradable macromolecule with a second electric property is prepared, and the first electric property is opposite to the second electric property. The second biodegradable macromolecule is chitosan or collagen, for example, and the second electric property is positive charge. In detail, the second biodegradable macromolecule was chitosan. 5g low-viscous chitosan was added into 495 ml de-ionized water with 5 ml glacial acetic acid and stirred by electromagnetic stirrer until the chitosan solution stays in a yellow and pellucid state. Next, the glacial acetic acid in the chitosan solution was removed by membrane dialysis, and the pH of the chitosan solution was about 6.5 after dialysis. Then, the chitosan solution was filtrated by air suction filter to remove the impurity. Finally, the chitosan solution was heated and stirred at 135°C for being concentrated until the concentration of the chitosan solution reaches 20-30 mg/ml, which was the second solution comprising the second biodegradable macromolecule with positive charge.

Similarly, one skilled in the art will readily recognize that the foregoing method of preparation of the second solution is one of embodiments. After reading and understanding the descriptions of the present invention, it will be obvious to those skilled in the art that various modifications may be made and not limited to the foregoing embodiment.

It is noted that the foregoing first and second biodegradable macromolecule can be natural macromolecules, such as heparin or polyglutamic acid, and chitosan or collgen, respectively. The foregoing first and second biodegradable macromolecule also can be artificially biodegradable macromolecules.

### Example 4: Form a biodegradable nanocomplex

The mixture solution was added into the second solution to form a biodegradable nanocomplex by attraction force between the different electric properties, and the dengue viral protein was held in the biodegradable nanocomplex. Fig. 1 is an electron microscope image of a biodegradable nanocomplex holding a dengue viral protein inside according to an embodiment of the present invention. It is worth noted that the dengue viral protein with negative charge is mixed with the polyglutamic acid solution with negative charge to form a mixture solution first, and then the mixture solution is mixed with the chitosan solution with positive charge to form the biodegradable nanocomplex solution, in which the structure of the biodegradable nanocomplex is more stable. However, the dengue viral protein with negative charge also can be mixed with the chitosan solution with positive charge first to form a mixture solution, and then the mixture solution is mixed with the polyglutamic acid solution. Because of the biodegradability of the first and second macromolecule, the nanocomplex formed from the first and second macromolecule has biodegradability as well. The biodegradability means that the nanocomplex is decomposed, absorbed and removed easily and naturally by the human body after it enters the human body, and the dengue viral protein held in the nanocomplex is released slowly for the sustained release. Table 1 is the particle size and the zeta potential of the biodegradable nanocomplex with and without holding the dengue viral protein. The charge ratio of the chitosan with positive charge to the polyglutamic acid with negative charge is preferably 4:1 because the structure of the biodegradable nanocomplex stays in a most stable state under this condition. The results of the particle size and zeta potential are the average value of three biodegradable nanocomplexes.

**Table 1**

| | without holding dengue viral protien | with holding dengue viral protein |
|---|---|---|
| Size (nm) | 126.4±5.1 | 124.5±1.8 |
| Zeta potential (mV) | 83.5±6.2 | 15.4±0.7 |

### Example 5: Vaccination with the biodegradable nanocomplex holding dengue viral protein inside on a mouse model

Mice are vaccinated with the biodegradable nanocomplex holding the dengue viral protein inside as a model compound. C3H/HeN mice were obtained from the Jackson Laboratory, and maintained on standard laboratory food and water in the Laboratory Animal Center of National Cheng Kung University Medical College in Taiwan (R.O.C.). Housing, breeding, and experimental use of the animals were performed in strict accordance with the Experimental Animal Committee in the laboratory animal center of National Cheng Kung University. Table 2 is the results of a titer of a neutralizing antibody in the mice vaccinated by the biodegradable nanocomplex holding the dengue viral protein inside of the present invention, by the traditional Alum adjuvant, or by the traditional Ribi adjuvant.

**Table 2**

| Vaccination time | Antibody titer to the DJ NS 1(x10³) | | | | | |
|---|---|---|---|---|---|---|
| | Nanocomplex (µg/mouse) | | Alum (µg/mouse) | | Ribi (µg/mouse) | |
| | 25 | 50 | 25 | 50 | 25 | 50 |
| First | ND | ND | ND | ND | ND | ND |
| Second | 2⁸ | 2⁹ | ND | ND | ND | ND |
| Third | 2¹⁰ | 2¹¹ | 2⁶ | 2⁸ | 2⁷ | 2⁸ |

According to Table 2, after vaccination twice, a specific antibody response was induced by the dengue vaccine comprising the biodegradable nanocomplex of the present invention, and the mice had the antibody titer of 1:256000 when the dose of the biodegradable nanocomplex in the dengue vaccine is 25 µg per vaccination. Accordingly, the vaccination times of the biodegradable high-efficiency dengue vaccine in the present invention is decreased, so the biodegradable high-efficiency dengue vaccine is good for being a commercial vaccine. The ND means that the antibody titer is non-detectable. The antibody titer was measured by an ELISA standard protocol. The time of antibody response induced by the biodegradable nanocomplex was faster than that induced by the traditional Alum adjuvant and Ribi adjuvant. In detail, after vaccination with the biodegradable nanocomplex twice, an organism has antibody responses, but the traditional Alum adjuvant and Ribi adjuvant induced the specific antibody response to the dengue viral protein in the mice until the third vaccination. In comparison with the Alum adjuvant and Ribi adjuvant used in the traditional dengue vaccine of the prior art, the vaccination times of the biodegradable high-efficiency dengue vaccine in the present invention is decreased to reduce the vaccination cost, so the biodegradable high-efficiency dengue vaccine is good for being a commercial vaccine. Moreover, because of the biodegradability of the nanocomplex, the dengue vaccine is decomposed, absorbed and removed easily and naturally by the human body after it enters the human body, after the third vaccination, the antibody titer induced by the biodegradable nanocomplex of the present invention was higher than that induced by the foregoing Alum adjuvant and Ribi adjuvant. It resolves the unsafe problem resulting from the heavy metal of the Alum adjuvant, and the dengue viral protein held in the biodegradable nanocomplex is released slowly for the sustained release. It is suggested that the biodegradable nanocomplex holding dengue viral protein inside enhanced the adjuvant effect in the dengue vaccine. The foregoing Ribi adjuvant was non-toxic and non-immunity oil-in-water emulsions in Ribi adjuvant system (RAS) developed by the Ribi Immunochem Research Inc. in 1985.

In the other embodiment of the present invention, the biodegradable nanocomplex made from heparin as the first biodegradable macromolecule and chitosan as the second biodegradable macromolecule also induced the specific antibody response to the dengue viral protein in mice after the second vaccination, and the dose of the biodegradable nanocomplex in the dengue vaccine is 25 µg per vaccination. The organism had the antibody titer of 1:32000 at least after the second vaccination.

A pharmaceutical composition comprising the dengue vaccine comprising the foregoing biodegradable nanocomplex is also provided, which is used for producing a vaccine or a drug for treating or preventing hemorrhagic dengue fever or dengue shock syndrome. The pharmaceutical composition comprises the foregoing biodegradable high-efficiency dengue vaccine or an addition salts thereof with a pharmaceutically acceptable base, and at least one pharmaceutically acceptable excipient. Moreover, the pharmaceutical composition of the present invention can be administered to animals in any existing ways, i.e. oral, nasal, mucosal, topical, dermal, and parenteral administration, wherein parenteral administration is intravenous, intraperitoneal, intradermal, subcutaneous, or intramuscular administration. The pharmaceutical composition of the present invention also can be administered via the combination of the foregoing administrations. For example, the first vaccination is via parenteral administration, and the second vaccination is via mucosal administration. In addition, the dose of the pharmaceutical composition varies depending on the species, age, weight, and status of individuals, the disease to be prevented or treated, the seriousness of the disease, the specific compound use in the pharmaceutical composition, and administration methods. One skilled in the art will readily recognize the publication content of the present invention, a proper dose can be decided by the routine experiment, and after the first vaccination, the organism can be decided to receive one or more additional vaccinations at a proper interval.

According to the above description, in comparison with the traditional technique, the biodegradable high-efficiency dengue vaccine, the method for making the same and the pharmaceutical composition of the same according to the present invention has the advantages as following:
1. The dengue vaccine comprising the biodegradable nanocomplex of the present invention can induce a specific antibody response to the dengue viral protein in mice after vaccination twice. In comparison with the Alum adjuvant and Ribi adjuvant used in the traditional dengue vaccine of the prior art, the vaccination times of the biodegradable high-efficiency dengue vaccine in the present invention is decreased to further reduce the vaccination cost, so the biodegradable high-efficiency dengue vaccine is good for being a commercial vaccine.
2. After vaccination with the dengue vaccine of the present invention twice, the organism has the antibody titer of 32000 at least. In comparison with the prior art, the dengue vaccine of the present invention substantially increases the antibody production to induce the immune response efficiently for enhancing the protection effect of the vaccine.
3. The biodegradable nanocomplex of the dengue vaccine of the present invention is made from the mix of the biodegradable polyglutamic acid (or heparin) and chitosan to hold the dengue viral protein inside. Accordingly, the dengue vaccine is decomposed, absorbed and removed easily and naturally by the human body after it enters the human body. It resolves the unsafe problems resulting from the heavy metal of the Alum adjuvant, and the dengue viral protein held in the nanocomplex is released slowly for the sustained release.

## Claims

1. A biodegradable high-efficiency dengue vaccine, comprising :
a biodegradable nanocomplex with an electric property holding a dengue viral protein inside, wherein the biodegradable nanocomplex is made from a chitosan with positive charge and a polyglutamic acid with negative charge and the biodegradable nanocomplex having an electric property of positive charge, wherein a charge ratio of the chitosan to the polyglutamic acid is 4:1, and
wherein the dengue viral protein comprises a nonstructural chimeric protein DJ NS1 of SEQ.ID. NO:1.

2. The biodegradable high-efficiency dengue vaccine as claimed in claim 1, wherein the dose of the biodegradable nanocomplex is no more than 25 µg for a first vaccination.

3. A method for making a biodegradable high-efficiency dengue vaccine, comprising the steps of:
adding a dengue viral protein into a first solution to form a mixture solution, followed by adding said mixture solution into a second solution to form a biodegradable nanocomplex with positive charge by attraction force between the different electric properties, wherein the first solution comprises a first biodegradable macromolecule with a first electric property that is the same as an electric property of the dengue viral protein, the first electric property is a negative charge, the first macromolecule is polyglutamic acid, the dengue viral protein has a nonstructural chimeric protein DJ NS1 of SEQ.ID. NO:1,
the second solution comprising a second biodegradable macromolecule with a second electric property that is opposite to the first electric property, wherein the second macromolecule is chitosan, wherein a charge ratio of the chitosan to the polyglutamic acid is 4:1, and
the dengue viral protein is held in the positively charged biodegradable nanocomplex.

4. A pharmaceutical composition comprising:
a biodegradable high-efficiency dengue vaccine as claimed in claim 1 or a salt thereof with a pharmaceutically acceptable base; and
at least one pharmaceutically acceptable excipient.

5. The pharmaceutical composition as claimed in claim 4 which is used for producing a drug for treating or preventing hemorrhagic dengue fever or dengue shock syndrome.

## Patentansprüche

1. Hocheffizienter biologisch abbaubarer Dengue - Impfstoff, umfassend:
einen biologisch abbaubaren Nanokomplex mit einer ein Dengue - Virusprotein im Inneren haltenden elektrischen Eigenschaft, wobei der biologisch abbaubare Nanokomplex aus einem Chitosan mit positiver Ladung und einer Polyglutaminsäure mit negativer Ladung hergestellt ist, und wobei der biologisch abbaubare Nanokomplex die elektrische Eigenschaft einer positiven Ladung hat, wobei ein Ladungsverhältnis von Chitosan zu Polyglutaminsäure 4:1 beträgt, und wobei das Dengue - Virusprotein ein nicht-strukturelles chimäres Protein DJ NS1 der SEQ. ID. NR.: 1 umfasst.

2. Hocheffizienter biologisch abbaubarer Dengue - Impfstoff nach Anspruch 1, bei welchem die Dosis des biologisch abbaubaren Nanokomplexes nicht höher als 25 µg für eine erste Impfung ist.

3. Verfahren zur Herstellung eines hocheffizienten biologisch abbaubaren Dengue - Impfstoffs, welches als Schritte umfasst:
Zugabe eines Dengue - Virusproteins in eine erste Lösung zur Ausbildung einer Mischungslösung, wobei nachfolgend die Mischungslösung in eine zweite Lösung hinzugegeben wird, um einen biologisch abbaubaren Nanokomplex mit positiver Ladung durch die Anziehungskraft zwischen den unterschiedlichen elektrischen Eigenschaften auszubilden, wobei die erste Lösung ein erstes biologisch abbaubares Makromolekül mit einer ersten elektrischen Eigenschaft beinhaltet, welche die gleiche ist wie eine elektrische Eigenschaft des Dengue - Virusproteins, wobei die erste elektrischen Eigenschaft eine negative Ladung ist, wobei das erste Makromolekül Polyglutaminsäure ist, wobei das Dengue - Virusprotein ein nicht-strukturelles chimäres Protein DJ NS1 mit der SEQ. ID. NR.:1 aufweist,
wobei die zweite Lösung ein zweites biologisch abbaubares Makromolekül mit einer zweiten elektrischen Eigenschaft umfasst, welche zu der ersten elektrischen Eigenschaft entgegengesetzt ist, wobei das zweite Makromolekül Chitosan ist, wobei ein Ladungsverhältnis von Chitosan zu Polyglutaminsäure 4:1 beträgt, und
wobei das Dengue - Virusprotein in dem positiv geladenen biologisch abbaubaren Nanokomplex gehalten wird.

4. Pharmazeutische Zusammensetzung, umfassend:
einen hocheffizienten biologisch abbaubaren Dengue - Impfstoff nach Anspruch 1 oder ein Salz davon mit einer pharmazeutisch verträglichen Basis, und wenigstens einen pharmazeutisch verträglichen Hilfsstoff.

5. Pharmazeutische Zusammensetzung nach Anspruch 4, welche zur Herstellung eines Medikaments zur Behandlung oder Verhinderung von hämorrhagischen Dengue-Fieber oder dem Dengue - Schock - Syndrom verwendet wird.

## Revendications

1. Vaccin biodégradable à haute efficacité contre la dengue, comprenant:
un nanocomplexe biodégradable avec une propriété électrique retenant une protéine virale de dengue à l'intérieur, dans lequel le nanocomplexe biodégradable est fait d'un chitosan avec une charge positive et un acide polyglutamique avec une charge négative et le nanocomplexe biodégradable possède une propriété électrique de charge positive, dans lequel un rapport de charge du chitosan par rapport à l'acide polyglutamique est 4:1, et dans lequel la protéine virale de dengue comprend une protéine chimérique non structurale DJ NS1 de SEQ. ID. n° : 1.

2. Vaccin biodégradable à haute efficacité contre la dengue selon la revendication 1, dans lequel la dose du nanocomplexe biodégradable n'est pas supérieure à 25 µg pour une première vaccination.

3. Procédé pour fabriquer un vaccin biodégradable à haute efficacité contre la dengue, comprenant les étapes de:
l'ajout d'une protéine virale de dengue dans une première solution pour former une solution de mélange, suivi par l'ajout de ladite solution de mélange dans une seconde solution pour former un nanocomplexe biodégradable avec une charge positive par force d'attraction entre les propriétés électriques différentes, dans lequel la première solution comprend une première macromolécule biodégradable avec une première propriété électrique qui est la même qu'une propriété électrique de la protéine virale de dengue, la première propriété électrique est une charge négative, la première macromolécule est de l'acide polyglutamique, la protéine virale de dengue possède une protéine chimérique non structurale DJ NS1 de SEQ. ID. n° : 1, la seconde solution comprenant une seconde macromolécule biodégradable avec une seconde propriété électrique qui est opposée à la première propriété électrique, dans lequel la seconde macromolécule est du chitosan, dans lequel un rapport de charge du chitosan par rapport à l'acide polyglutamique est 4:1, et
la protéine virale de dengue est retenue dans le nanocomplexe biodégradable à charge positive.

4. Composition pharmaceutique, comprenant:
un vaccin biodégradable à haute efficacité contre la dengue selon la revendication 1 ou un sel de celui-ci avec une base pharmaceutiquement acceptable; et au moins un excipient pharmaceutiquement acceptable.

5. Composition pharmaceutique selon la revendication 4, qui est utilisé pour produire un médicament pour traiter ou empêcher la fièvre de dengue hémorragique ou le syndrome de choc de dengue.
